# EUROPEAN PATENT SPECIFICATION

(11) **EP 2 114 314 B1**
(45) Date of publication and mention of the grant of the patent: **24.04.2019**
(21) Application number: 08713431.8
(22) Date of filing: 03.01.2008
(51) Int. Cl.: A61F 2/44

(54) **SPINAL PROSTHESIS SYSTEMS**
WIRBELSÄULENPROTHESESYSTEME
SYSTÈMES DE PROTHÈSE VERTÉBRALE

(30) Priority: 03.01.2007 US 619340
(43) Date of publication of application: 11.11.2009
(73) Proprietor: Warsaw Orthopedic, Inc., Warsaw, IN 46581 (US)
(72) Inventor: ZHANG, Zhilong, Collierville, Minnesota 38017 (US); ALLARD, Randall N., Issaquah, WA 98029 (US); CARLS, Thomas A., Memphis, Tennessee 38103 (US); MURRELL, Charles B., Olive Branch, Tennessee 38654 (US)
(74) Representative: Viering, Jentschura & Partner mbB Patent- und Rechtsanwälte
(86) International application number: PCT/US2008/050073
(87) International publication number: WO 2008/086072

(56) References cited:
- WO-A-2007/075411
- US-A1- 2002 049 497
- US-A1- 2004 225 365
- US-A1- 2005 222 683
- US-A1- 2006 041 313
- US-A1- 2006 069 440
- US-A1- 2006 136 063
- US-A1- 2006 155 377

## Description

### TECHNICAL FIELD

Embodiments of the present disclosure relate generally to devices and methods for spinal surgery, and more particularly in some embodiments, to motion-preserving prosthetic devices having projections for engaging with the adjacent bone structure.

### BACKGROUND

US 2005/0222683 A1 discloses a disc replacement device comprising an upper shell, a lower shell, and plurality of compressible pillars each coupled to and connecting the upper and lower shells and comprising a shape memory alloy, wherein at least one of the plurality of pillars is interiorly offset from perimeters of the upper and lower shells.

US 2006/0069440 A1 discloses an implant for relieving pain associated with at least one of the spinal column and surrounding tissues and structures, which implant is positionable between a first vertebrae and a second vertebrae of the spinal column, wherein the first vertebrae is located adjacent to and above the second vertebra, the implant comprising a first end plate having a first support surface and a top surface opposite the first support surface wherein the first end plate comprises two or more first segments that are joined side by side; a second end plate having a second support surface wherein the second end plate comprises two or more second segments that are joined side by side; and a bearing member that is interposed between the first end plate and the second end plate wherein the bearing member has (i) a convex upper surface that is in contact with the first support surface and (ii) an opposite mounting surface that is in contact with the second support surface, wherein the top surfaces includes means for securing the first end plate to the first vertebra and wherein the lower surface includes means for securing the second end plate to the second vertebra, wherein the means for securing the first and second end plates to the first and second, respectively, vertebrae comprise first and second, respectively, keels emanating from the top and lower, respectively, surfaces of the first and second, respectively, end plates, and wherein the first and second keels extend into first and second, respectively, cavities formed in the first vertebra.

Although existing devices and methods have been generally adequate for their intended purposes, they have not been entirely satisfactory in all respects.

### SUMMARY

The invention provides intervertebral articulating prosthetic devices according to claim 1 and claim 8, respectively. Further embodiments of the invention are described in the dependent claims.

Additional and alternative features, advantages, uses, and embodiments are set forth in or will be apparent from the following description, drawings, and claims.

### Brief Description of the Drawings

The embodiments of Figs. 8-39 as such do not form embodiments of the invention.
Figure 1 is a diagrammatic lateral view of a pair of vertebrae.
Figure 2 is a diagrammatic perspective view of a prosthetic device that embodies aspects of the present disclosure.
Figure 3 is a partial cross-sectional front view of the prosthetic device of Figure 2.
Figure 4 is a side view of the prosthetic device of Figure 2.
Figure 5 is diagrammatic perspective view of a prosthetic device similar in some aspects to a portion of the prosthetic device of Figure 2, but showing an alternative embodiment.
Figure 6 is a front view of the prosthetic device of Figure 5.
Figure 7 is a side view of the prosthetic device of Figure 5.
Figure 8 is a diagrammatic perspective view of a prosthetic device similar in some aspects to the prosthetic device of Figure 2, but showing an alternative embodiment.
Figure 9 is a front view of the prosthetic device of Figure 8.
Figure 10 is a side view of the prosthetic device of Figure 8.
Figure 11 is a diagrammatic perspective view of a prosthetic device similar in some aspects to the prosthetic devices of Figures 2 and 8, but showing an alternative embodiment.
Figure 12 is a front view of the prosthetic device of Figure 11.
Figure 13 is a side view of the prosthetic device of Figure 11.
Figure 14 is a diagrammatic perspective view of a prosthetic device similar in some aspects to the prosthetic devices of Figures 2, 8, and 11, but showing an alternative embodiment.
Figure 15 is a front view of the prosthetic device of Figure 14.
Figure 16 is a side view of the prosthetic device of Figure 14.
Figure 17 is a bottom view of the prosthetic device of Figure 14.
Figure 18 is a diagrammatic perspective view of a prosthetic device similar in some aspects to the prosthetic devices of Figures 2, 8, 11, and 14, but showing an alternative embodiment.
Figure 19 is a perspective bottom view of the prosthetic device of Figure 18.
Figure 20 is a front view of the prosthetic device of Figure 18.
Figure 21 is a side view of the prosthetic device of Figure 18.
Figure 22 is a diagrammatic perspective view of a prosthetic device similar in some aspects to the prosthetic devices of Figures 2, 8, 11, 14, and 18, but showing an alternative embodiment.
Figure 23 is a front view of the prosthetic device of Figure 22.
Figure 24 is a diagrammatic perspective view of a prosthetic device similar in some aspects to the prosthetic devices of Figures 2, 8, 11, 14, 18, and 22, but showing an alternative embodiment.
Figure 25 is a front view of the prosthetic device of Figure 24.
Figure 26 is a side view of the prosthetic device of Figure 24.
Figure 27 is a diagrammatic perspective view of a prosthetic device similar in some aspects to the prosthetic devices of Figures 2, 8, 11, 14, 18, 22, and 24, but showing an alternative embodiment.
Figure 28 is a front view of the prosthetic device of Figure 27.
Figure 29 is a side view of the prosthetic device of Figure 27.
Figure 30 is a diagrammatic perspective view of a prosthetic device similar in some aspects to a portion of the prosthetic devices of Figures 2, 8, 11, 14, 18, 22, 24, and 27, but showing an alternative embodiment.
Figure 31 is a front view of the prosthetic device of Figure 30.
Figure 32 is a side view of the prosthetic device of Figure 30.
Figure 33 is a bottom view of the prosthetic device of Figure 30.
Figure 34 is a diagrammatic perspective view of a prosthetic device similar in some aspects to a portion of the prosthetic devices of Figures 2, 8, 11, 14, 18, 22, 24, 27, and 30, but showing an alternative embodiment.
Figure 35 is a diagrammatic perspective view of a prosthetic device similar in some aspects to a portion of the prosthetic devices of Figures 2, 8, 11, 14, 18, 22, 24, 27, 30, and 34, but showing an alternative embodiment.
Figure 36 is a diagrammatic perspective view of a prosthetic device similar in some aspects to a portion of the prosthetic devices of Figures 2, 8, 11, 14, 18, 22, 24, 27, 30, 34, and 35, but showing an alternative embodiment.
Figure 37 is a diagrammatic perspective view of a prosthetic device similar in some aspects to the prosthetic devices of Figures 2, 8, 11, 14, 18, 22, 24, and 27, but showing an alternative embodiment.
Figure 38 is a front view of the prosthetic device of Figure 37.
Figure 39 is a bottom view of the prosthetic device of Figure 37.

### DESCRIPTION

For the purposes of promoting an understanding of the principles of the invention, reference will now be made to the embodiments, or examples, illustrated in the drawings and specific language will be used to describe the embodiments. It will nevertheless be understood that no limitation of the scope of the invention is intended. Any alterations and further modifications of the described embodiments, and any further applications of the principles of the invention as described herein are contemplated as would normally occur to one skilled in the art to which the invention relates.

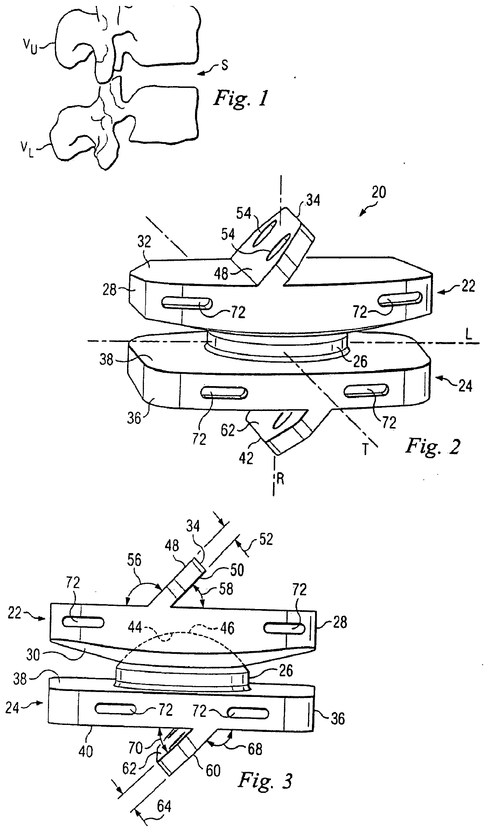

Figure 1 is a diagrammatic lateral view of a pair of vertebrae, an upper vertebra V_{U} and a lower vertebra V_{L}. The vertebrae V_{U}, V_{L} may be located in any region of the spine, including the cervical, thoracic, lumbar, and sacral regions. In that regard, the lower vertebra V_{L} is the sacrum in some instances. In some spinal surgeries, some or all of the natural disc that would have been positioned between the two vertebrae V_{U}, V_{L} is removed via a discectomy or a similar surgical procedure, the details of which would be known to one of ordinary skill in the art. Removal of the diseased or degenerated disc results in the formation of an intervertebral space S between the upper and lower vertebrae V_{U}, V_{L}. In some embodiments, the prosthetic devices described below are adapted for use and disposition within the intervertebral space S.

For simplicity the prosthetic devices and corresponding engagement structures are described below as being adapted for use with an anterior surgical approach. However, in other embodiments the shape of the prosthetic devices and the positioning of the keel structures thereon are configured for other surgical approaches including, but not limited to posterior, lateral, oblique surgical approaches, and combinations thereof. Thus, the prosthetic devices and concepts described herein are adaptable for use in the surgical approach that will be most beneficial to the patient.

Further, for the sake of brevity the various embodiments of prosthetic devices and corresponding engagement structures are described below with reference to particular exemplary combinations of components, features, and structures. However, it is understood that the various components, features, and structures of the exemplary embodiments are combinable in numerous other ways. Thus, features from one embodiment may be combined with features from another embodiment to form yet another embodiment of a prosthetic device according to the present disclosure even though such a combination is not explicitly shown. In particular, the various engagement structures described below may be used alone or in various combinations with the other engagement structures on a prosthetic device according to the present disclosure.

Referring to Figures 2-4, shown therein is an intervertebral articulating prosthetic device 20 according to one embodiment of the present disclosure. The prosthetic device 20 extends generally along a longitudinal axis L and includes an upper component 22, a lower component 24, and an articulating central portion 26. The upper and lower components 22, 24 and the articulating portion 26 cooperate to form the articulating device 20, which is sized and configured for disposition within an intervertebral space between a pair of vertebral bodies, such as the intervertebral space S between the adjacent vertebral bodies V_{U}, V_{L}. The upper component 22 includes a support plate 28 having an articulating surface 30 and an opposing bearing surface 32. An engagement structure 34 extends from the bearing surface 32. Similarly, the lower component 24 includes a support plate 36 having an articulating surface 38 and an opposing bearing surface 40. An engagement structure 42 extends from the bearing surface 40.

The prosthetic device 20 and, in particular, the articulating portion 26 provide relative pivotal, rotational, and/or translational movement between the adjacent vertebral bodies V_{U}, V_{L} to maintain or restore motion substantially similar to the normal biomechanical motion provided by a natural intervertebral disc. In some embodiments the articulating portion 26 comprises a ball-and-socket joint formed by the upper and lower components 22, 24. In one such embodiment shown in Figure 3, the articulating surface 30 of the upper component 22 includes an a recess 44 adapted to pivotally mate with a projection 46 extending from the articulating surface 38 of the lower component 24. Together the recess 44 and projection 46 form the ball-and-socket joint of the articulating portion 26. In this manner, the upper and lower components 22, 24 are configured to permit pivotal motion about a number of axes, including lateral or side-to-side pivotal movement about longitudinal axis L, anterior-posterior pivotal movement about a transverse axis T, rotational pivotal movement about a rotational axis R, and combinations thereof. In some embodiments, the recess 44 is shaped (e.g. with a larger radius of curvature than the projection 46) to allow translational movement along the longitudinal axis L, the transverse axis T, and combinations thereof.

Although the prosthetic device 20 and the articulating portion 26 have been illustrated and described as providing a specific combination of articulating motion, it should be understood that other combinations of articulating movement are also possible and are contemplated as falling within the scope of the present disclosure. In addition, the articulating motion may be biased towards a particular direction and/or limited in a particular direction. Further, the prosthetic device 20 may include stops to totally prevent motion in a certain direction past a predetermined threshold. Further, in some embodiments the articulating portion 26 is a completely separate component of the prosthetic device 20. That is, the articulating portion 26 is not formed by portions of the upper and lower components 22, 24, but rather is connected to, mated with, or otherwise oriented with the upper and lower components to provide at least some motion to the prosthetic device 20. In one such embodiment, the articulating portion 26 is formed of a resilient, elastic material.

In the current embodiment, the support plates 28, 36 are sized and shaped to substantially correspond to the size and shape of the vertebral endplate of an adjacent vertebra V_{U}, V_{L}. In other embodiments, the support plates 28, 36 are sized and shaped to correspond to only a portion of the vertebral endplate of the adjacent vertebra. For example, in one alternative embodiment the support plates 28, 36 are sized and shaped for use in a bilateral procedure. In some embodiments, the upper support plate 28 and the lower support plate 36 have different sizes.

The support plate 28 includes articulating surface 30. Referring more specifically to Figure 3, the articulating surface 30 includes the recess 44 as described above. In the current embodiment, the recess 44 is substantially shaped as a spherical socket. However, it should be understood that in other configurations the recess 44 has other shapes, such as, for example, cylindrical, elliptical, other arcuate configurations, and/or non-arcuate configurations. The recess 44 is shaped to mate with the projection 46 of the lower component 24 to provide at least some motion to the prosthetic device 20. The remaining portion of the articulating surface 30 is contoured to further facilitate the motion-preserving features of the prosthetic device 20 in some embodiments. For example, in the current embodiment the remaining portion of the articulating surface 30 is angled or sloped as it extends from the edge of the recess 44 to the edges of the articulating surface 30 to allow for a greater range of motion than would be possible if the remaining portion of the articulating surface was planar. In other embodiments, the remaining portion of the articulating surface 30 is substantially planar, includes stop portions, is angled in one direction but not another direction, and/or is otherwise configured to facilitate motion-preserving features, the insertion, and/or the general use of the prosthetic device 20.

Further, although the recess 44 is illustrated as having a generally smooth, uninterrupted surface, it should be understood that a surface depression, cavity, or groove may be defined along a portion of the recess to aid in clearing out matter, such as particulate debris, that is disposed between the abutting articulating surfaces 30, 38 of upper and lower components 22, 24. In one such embodiment, the surface of the projection 46 defines a generally smooth, uninterrupted articular surface. In another embodiment, each of the recess 44 and the convex projection 30 may define a surface depression to facilitate removal of particulate matter disposed between the abutting articulating surfaces 30, 38.

Referring again to Figures 2-4, the support plate 28 also includes bearing surface 32 with the engagement structure 34 extending therefrom. In the current embodiment, the engagement structure 34 comprises a single flange member or keel that extends substantially across the bearing surface 32 in a direction substantially transverse to the longitudinal axis L. The engagement structure 34 is configured for disposition within a preformed opening in the adjacent vertebral endplate. The engagement structure 34 includes opposing surfaces 48 and 50 separated by a width 52. In the current embodiment, the surfaces 48 and 50 are substantially planar and the width 52 is substantially constant along the length and height of the engagement structure 34. However, in other embodiments the width 52 varies along the length and/or height of the engagement structure 34. For example, in some embodiments the width 52 is narrowed towards at least one end and/or the upper portion of the engagement structure 34 compared to the other portions of the structure. In some embodiments, the width is narrowed such that the engagement structure 34 is capable of cutting into the bone structure to facilitate engagement of the prosthetic device 20 with the adjacent vertebrae V_{U}, V_{L}. Further, in some embodiments the engagement structure 34 is sharp enough that it is capable of being inserted without a preformed opening in the adjacent vertebrae. In some embodiments, the width varies such that the surfaces 48 and 50 are non-planar. In general the width 52 of the engagement structure is within a range of .5 mm to 10 mm, but may be smaller or greater in some embodiments.

The engagement structure 34 also includes a pair of openings 54 extending therethrough between the opposing surfaces 48, 50 to facilitate bone in-growth to enhance fixation to the adjacent vertebra. However, it should be understood that any number of openings may be defined through the engagement structure 34, including a single opening or three or more openings. It should also be understood that the openings 54 need not necessarily extend entirely through the engagement structure 34, but may alternatively extend partially therethrough. It should further be understood that the engagement structure 34 need not necessarily define any openings extending either partially or entirely therethrough. Additionally, although the openings 54 are illustrated as having a circular configuration, it should be understood that other shapes, sizes, and configurations of the openings are also contemplated.

In the current embodiment, the engagement structure 34 extends from the bearing surface 32 at an oblique angle. In particular, in the current embodiment the surface 48 extends from the bearing surface 32 at an angle 56, which is approximately 135°, and the surface 50 extends from the bearing surface at an angle 58, which is approximately 45°. Thus, as shown the surfaces 48 and 50 are substantially parallel to one another and the engagement structure 34 generally extends from the bearing surface 32 at approximately a 45° angle. In other embodiments, each of the angles 56 and 58 have other values within the range of 10° to 170° such that the surfaces 48 and 50 are substantially parallel. In yet other embodiments, each of the angles 56 and 58 have values within the range of 10° and 170°, but such that the surfaces 48 and 50 are not substantially parallel. The precise choice of angles 56, 58 can be adapted for the particular patient and/or application. In some embodiments, the engagement structure 34 is substantially perpendicular to the bearing surface 32. In the current embodiment, the engagement structure 34 as a whole is approximately centrally located along the longitudinal axis L of the prosthetic device 20, as best seen in Figure 3.

The portions of bearing surface 32 and engagement structure 34 that are in direct contact with vertebral bone are coated with a bone-growth promoting substance in some embodiments. For example, in one aspect the bearing surface 32 and the engagement structure 34 are coated with a bone-growth promoting substance to promote bony engagement with the adjacent vertebra. Further, in some embodiments the openings 54 may be filled with a bone-growth promoting substance to further enhance bone in-growth. Also, the bearing surface 32 and engagement structure 34 can be roughened in lieu of or prior to application of the bone-growth promoting surface.

The support plate 36 of the lower component 24 includes the articulating surface 38. The articulating surface 38 includes the projection 46 having a convex shape. As shown in Figure 3, the projection 46 is configured as a portion of a spherical-shaped ball in some embodiments. It should be understood that other configurations of the projection 46 are utilized in other embodiments, such as, for example, cylindrical, elliptical or other arcuate configurations or possibly non-arcuate configurations. The remaining portion of the articulating surface 38 is substantially planar in the current embodiment. However, it should be understood that the remaining portion of articulating surface 38 non-planar or non-planar/planar combination configurations in other embodiments. For example, in some embodiments the articulating surface 38 includes an angular or conical configuration extending about the projection 46. Further, in some embodiments the surface of the projection 46 is interrupted by a surface depression, cavity, groove, or other interruption extending along the projection. The interruption facilitates the removal of matter disposed between abutting portions of the upper and lower components 22, 24 forming the articulating portion 26. More specifically, the interruption aids in clearing out matter such as, for example, particulate material, that is disposed between the abutting articulating surfaces 30, 38 of the upper and lower components 22, 24.

Referring again to Figures 2-4, the support plate 32 also includes the bearing surface 40 with the engagement structure 42 extending therefrom. In the current embodiment, the engagement structure 42 is substantially similar to the engagement structure 34 of the upper component 22. The engagement structure 42 comprises a single flange member or keel that extends substantially across the bearing surface 32 in a direction substantially transverse to the longitudinal axis L. The engagement structure 42 is configured for disposition within a preformed opening in the adjacent vertebral endplate. The engagement structure 42 includes opposing surfaces 60 and 62 separated by a width 64. In the current embodiment, the surfaces 60 and 62 are substantially planar and the width 64 is substantially constant along the length and height of the engagement structure 42 and is substantially equal to the width 52 of the engagement structure 34 of the upper component 22. However, in other embodiments the width 64 varies along the length and/or height of the engagement structure 42. For example, in some embodiments the width 64 is narrowed towards at least one end and/or the upper portion of the engagement structure 42 compared to the other portions of the structure. In some embodiments, the width is narrowed such that the engagement structure 42 is capable of cutting into the bone structure to further facilitate engagement of the prosthetic device 20 with the adjacent vertebrae V_{U}, V_{L}. Further, in some embodiments the engagement structure 34 is sharp enough that it is capable of being inserted without a preformed opening in the adjacent vertebrae. In some embodiments, the width varies such that the surfaces 60 and 62 are non-planar. Also, the width 64 of the engagement structure 42 is different from the width 52 of the engagement structure 34 in some embodiments. In general, the width 64 of the engagement structure is within a range of .5 mm to 10 mm, but may be smaller or greater in some embodiments.

The engagement structure 42 also includes a pair of openings 66 extending therethrough between the opposing surfaces 60, 62 to facilitate bone in-growth to enhance fixation to the adjacent vertebra. However, it should be understood that any number of openings may be defined through the engagement structure 42, including a single opening or three or more openings. It should also be understood that the openings 66 need not necessarily extend entirely through the engagement structure 42, but may alternatively extend partially therethrough. It should further be understood that the engagement structure 42 need not necessarily define any openings extending either partially or entirely therethrough. Additionally, although the openings 66 are illustrated as having a circular configuration, it should be understood that other shapes, sizes, and configurations of the openings are also contemplated.

In the current embodiment, the engagement structure 42 extends from the bearing surface 40 at an oblique angle. In particular, in the current embodiment the surface 60 extends from the bearing surface 32 at an angle 68, which is approximately 135°, and the surface 62 extends from the bearing surface at an angle 70, which is approximately 45°. Thus, as shown the surfaces 60 and 62 are substantially parallel to one another and the engagement structure 42 generally extends from the bearing surface 40 at approximately a 45° angle. In other embodiments, each of the angles 68 and 70 have other values within the range of 10° to 170° such that the surfaces 60 and 62 are substantially parallel. In yet other embodiments, each of the angles 68 and 70 have values within the range of 10° and 170°, but such that the surfaces 60 and 62 are not substantially parallel. In some embodiments, the engagement structure 42 is substantially perpendicular to the bearing surface 40. The precise choice of angles 68, 70 can be adapted for the particular patient and/or application. Further, the angles 68, 70 may be selected for a particular orientation of the engagement structure 42 with respect to the engagement structure 34.

As shown in Figure 3, when the prosthetic device 20 is in a neutral position the engagement structure 42 extends in a direction substantially parallel to the engagement structure 34 of the upper component 22. In other embodiments, the engagement structure 42 is at an angle with respect to the engagement structure 34 when the prosthetic device 20 is in a neutral position. In some embodiments, the engagement structure 42 is substantially perpendicular to the engagement structure 34. In yet other embodiments, the engagement structure 42 is substantially aligned with the engagement structure 34.

In the current embodiment, the engagement structure 42 as a whole is approximately centrally located along the longitudinal axis L of the prosthetic device 20.

The portions of bearing surface 40 and engagement structure 42 that arc in direct contact with vertebral bone are coated with a bone-growth promoting substance in some embodiments. For example, in one aspect the bearing surface 40 and the engagement structure 42 are coated with a bone-growth promoting substance to promote bony engagement with the adjacent vertebra. Further, in some embodiments the openings 66 may be filled with a bone-growth promoting substance to further enhance bone in-growth. Also, the bearing surface 40 and engagement structure 42 can be roughened in lieu of or prior to application of the bone-growth promoting surface.

Referring again to Figures 2-4, in some embodiments the prosthetic device 20 includes one or more notches 72, or other types of structure, for receiving and engaging with a corresponding portion of a surgical instrument (not shown) to aid in the manipulation and insertion of the prosthetic device 20 within the intervertebral space between the adjacent vertebrae. The surgical instrument (not shown) is preferably configured to hold the upper and lower components 22, 24 at a predetermined orientation and spatial relationship relative to one another during the manipulation and insertion of the prosthetic device 20, and to release the upper and lower components once properly positioned between the adjacent vertebrae.

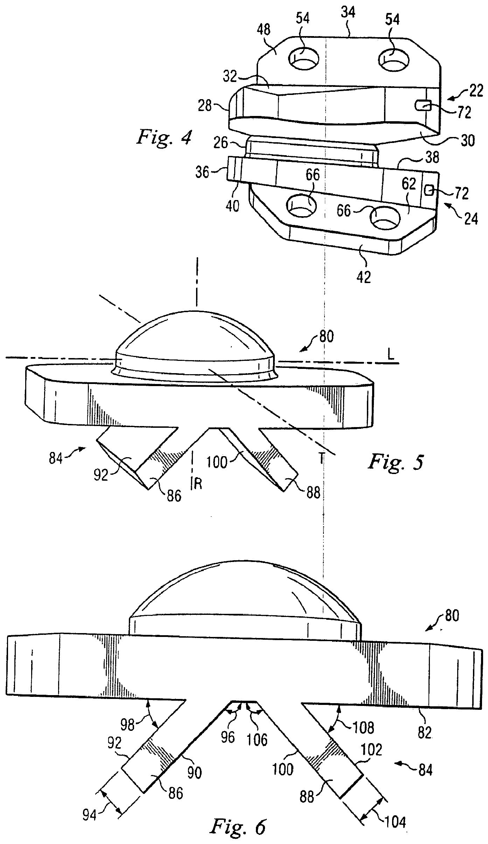

Referring now to Figures 5-7, shown therein is a component 80 that is an alternative embodiment of the upper and/or lower components 22, 24 of the prosthetic device 20 described above. As the component 80 is substantially similar in some aspects to the upper and lower components 22, 24 described above, only portions of the component 80 will be described in detail. The component 80 includes a bearing surface 82 that is substantially planar. An engagement structure 84 extends from the bearing surface 82. The engagement structure 84 is one example of an alternative to the engagement structures 34, 42 described above.

The engagement structure 84 includes a pair of flange members or keels 86 and 88. Each of the flange members 86, 88 extend substantially across the bearing surface 82 in a direction substantially transverse to the longitudinal axis L. The engagement structure flange members 86, 88 are configured for disposition within preformed openings in the adjacent vertebral endplate. The flange member 86 includes opposing surfaces 90 and 92 separated by a width 94. In the current embodiment, the width 94 is substantially uniform along the length and height of the flange member 86. However, in other embodiments the width 94 varies along the length and/or height of the flange member 86. For example, in some embodiments the width 94 is narrowed towards at least one end and/or the upper portion of the flange member 86 compared to the other portions of the member. In some embodiments, the width 94 is narrowed such that the flange member 86 is capable of cutting into the bone structure to further facilitate engagement of the prosthetic device 20 with the adjacent vertebrae V_{U}, V_{L}. Further, in some embodiments the engagement structure 34 is sharp enough that it is capable of being inserted without a preformed opening in the adjacent vertebrae. In some embodiments, the width 94 varies such that the surfaces 90 and 92 are non-planar. In general the width 94 of the flange member is within a range of .5 mm to 10 mm, but may be smaller or greater in some embodiments.

In the current embodiment, the flange member 86 extends from the bearing surface 82 at an oblique angle. In particular, the surface 90 extends from the bearing surface 32 at an angle 96, which is approximately 135°, and the surface 92 extends from the bearing surface at an angle 98, which is approximately 45°. Thus, as shown the surfaces 90 and 92 are substantially parallel to one another and the flange member 86 generally extends from the bearing surface 82 at approximately a 45° angle. In other embodiments, each of the angles 96 and 98 have other values within the range of 10° to 170° such that the surfaces 90 and 92 are substantially parallel. In yet other embodiments, each of the angles 96 and 98 have values within the range of 10° and 170°, but such that the surfaces 90 and 92 are not substantially parallel. The precise choice of angles can be adapted for the particular patient and/or application. In some embodiments, the flange member 86 is substantially perpendicular to the bearing surface 82.

The flange member 88 includes opposing surfaces 100 and 102 separated by a width 104. In the current embodiment, the width 104 is substantially uniform along the length and height of the flange member 86 and is substantially equal to the width 94 of the flange member 86. However, in other embodiments the width 104 varies along the length and/or height of the flange member 88. For example, in some embodiments the width 104 is narrowed towards at least one end and/or the upper portion of the flange member 88 compared to the other portions of the member. In some embodiments, the width 104 is narrowed such that the flange member 88 is capable of cutting into the bone structure to further facilitate engagement of the component 80 with the adjacent vertebrae V_{U}, V_{L}. Further, in some embodiments the engagement structure 34 is sharp enough that it is capable of being inserted without a preformed opening in the adjacent vertebrae. In some embodiments, the width 104 varies such that the surfaces 100 and 102 are non-planar. Also, the width 104 of the flange member 88 is different from the width 94 of the flange member 86 in some embodiments. In general the width 104 of the flange member is within a range of .5 mm to 10 mm, but may be smaller or greater in some embodiments.

In the current embodiment, the flange member 88 extends from the bearing surface 82 at an oblique angle. In particular, the surface 100 extends from the bearing surface 82 at an angle 106, which is approximately 135°, and the surface 102 extends from the bearing surface at an angle 108, which is approximately 45°. Thus, as shown the surfaces 100 and 102 are substantially parallel to one another and the flange member 88 generally extends from the bearing surface 82 at approximately a 45° angle. Thus, the flange member 88 is substantially perpendicular to the flange member 86. In other embodiments, each of the angles 106 and 108 have other values within the range of 10° to 170° such that the surfaces 100 and 102 are substantially parallel. In yet other embodiments, each of the angles 106 and 108 have values within the range of 10° and 170°, but such that the surfaces 100 and 102 are not substantially parallel. In some embodiments, the flange member 88 is substantially perpendicular to the bearing surface 82. The precise choice of angles can be adapted for the particular patient and/or application. Further, the angles 106, 108 may be selected for a particular orientation of the flange member 88 with respect to the flange member 86. That is, in some embodiments the flange members 86, 88 are not substantially perpendicular to one another, but are otherwise oriented with respect to one another including parallel and angular relationships.

In the current embodiment, the engagement structure 84 as a whole is approximately centrally located along the longitudinal axis L of the component 80 with the flange members 86, 88 spaced symmetrically about a midpoint of the component, as best seen in Figure 6.

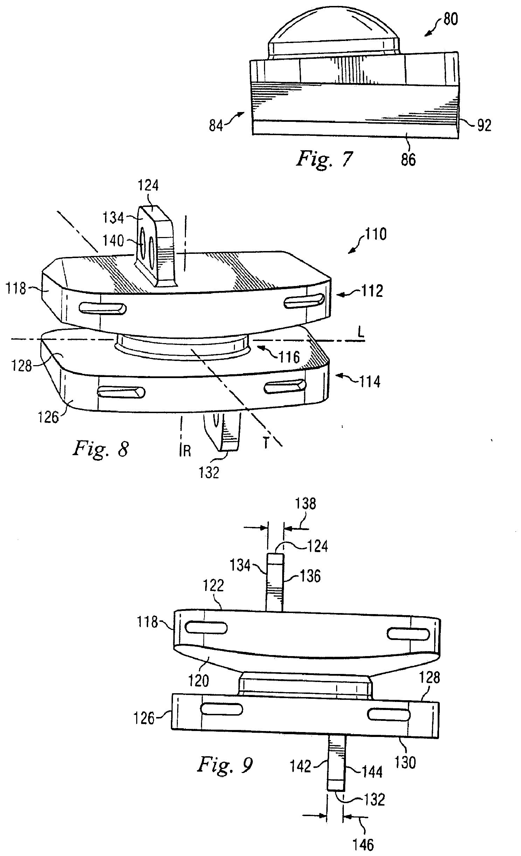

Referring to Figures 8-10, shown therein is another embodiment of a prosthetic device 110 according to another aspect of the present disclosure. The prosthetic device 110 is an alternative to the prosthetic device 20 described above. As the prosthetic device 110 is substantially similar in some aspects to the prosthetic device 20 described above, only portions of the prosthetic device 110 will be described in detail. The prosthetic device 110 extends generally along a longitudinal axis L and includes an upper component 112, a lower component 114, and an articulating central portion 116. The upper and lower components 112, 114 and the articulating portion 116 cooperate to form the articulating device 110, which is sized and configured for disposition within an intervertebral space between a pair of vertebral bodies, such as the intervertebral space S between the adjacent vertebral bodies V_{U}, V_{L}.

The upper component 112 includes a plate 118 having an articulating surface 120 and an opposing engagement surface 122. An engagement structure 124 extends from the engagement surface 122. Similarly, the lower component 114 includes a plate 126 having an articulating surface 128 and an opposing engagement surface 130. An engagement structure 132 extends from the bearing surface 130.

In the current embodiment, the plates 118, 126 are sized and shaped to substantially correspond to the size and shape of the vertebral endplate of an adjacent vertebra V_{U}, V_{L}. In other embodiments, the plates 118, 126 are sized and shaped to correspond to only a portion of the vertebral endplate of the adjacent vertebra. For example, in one alternative embodiment the plates 118, 126 are sized and shaped for use in a bilateral procedure. In some embodiments, the plate 28 and the plate 36 have different sizes.

The engagement structure 124 comprises a single flange member or keel that extends across a portion of the engagement surface 122 in a direction substantially transverse to the longitudinal axis L. In the current embodiment, the engagement structure 124 is substantially transverse to the engagement surface 122. However, in other embodiments the engagement structure 124 extends from the engagement surface 122 at an oblique angle. The engagement structure 124 is offset along the longitudinal axis L from the center point of the prosthetic device 110, as best seen in Figure 9. In other embodiments, the engagement structure 124 is positioned centrally along the longitudinal axis L. In yet other embodiments, the engagement structure 124 is located in other positions and orientations, including central and non-central locations relative to the longitudinal axis L and/or the transverse axis T.

Referring again to Figures 8-10, the engagement structure 124 is configured for disposition within a preformed opening in the adjacent vertebral endplate. The engagement structure 124 includes opposing surfaces 134 and 136 separated by a width 138. In the current embodiment, the surfaces 134 and 136 are substantially planar and the width 138 is substantially constant along the length and height of the engagement structure 124. However, in other embodiments the width 138 varies along the length and/or height of the engagement structure 124. For example, in some embodiments the width 138 is narrowed towards at least one end and/or the upper portion of the engagement structure 124 compared to the other portions of the structure. In some embodiments, the width 138 is narrowed such that the engagement structure 124 is capable of cutting into the bone structure to facilitate engagement of the prosthetic device 110 with the adjacent vertebrae V_{U}, V_{L}. Further, in some embodiments the engagement structure 124 is sharp enough that it is capable of being inserted without a preformed opening in the adjacent vertebrae. In some embodiments, the width varies such that the surfaces 134 and 136 are non-planar. In general the width 138 of the engagement structure 124 is within a range of .5 mm to 10 mm, but may be smaller or greater in some embodiments.

The engagement structure 124 also includes a pair of openings 140 extending therethrough between the opposing surfaces 134, 136 to facilitate bone in-growth to enhance fixation to the adjacent vertebra. However, it should be understood that any number of openings may be defined through the engagement structure 124, including a single opening or three or more openings. It should also be understood that the openings 140 need not necessarily extend entirely through the engagement structure 124, but may alternatively extend partially therethrough. It should further be understood that the engagement structure 124 need not necessarily define any openings extending either partially or entirely therethrough. Additionally, although the openings 140 are illustrated as having a circular configuration, it should be understood that other shapes, sizes, and configurations of the openings are also contemplated.

The engagement structure 132 is substantially similar to the engagement structure 124 of the upper component 112. The engagement structure 132 comprises a single flange member or keel that extends across a portion of the engagement surface 130 in a direction substantially transverse to the longitudinal axis L and offset from the engagement structure 124 of the upper component 112. Further, in the current embodiment the engagement structure 132 is substantially transverse to the engagement surface 130. However, in other embodiments the engagement structure 132 extends from the engagement surface 130 at an oblique angle. The engagement structure 132 is offset along the longitudinal axis L from the center point of the prosthetic device 110, as best seen in Figure 9. In other embodiments, the engagement structure 132 is positioned centrally along the longitudinal axis L. In yet other embodiments, the engagement structure 132 is located in other positions and orientations, including central and non-central locations relative to the longitudinal axis L and/or the transverse axis T and including locations substantially aligned and offset with respect to the engagement structure 124.

The engagement structure 132 is configured for disposition within a preformed opening in the adjacent vertebral endplate. The engagement structure 132 includes opposing surfaces 142 and 144 separated by a width 146. In the current embodiment, the surfaces 142 and 144 are substantially planar and the width 146 is substantially constant along the length and height of the engagement structure 132 and substantially equal to the width 138 of the engagement structure 124 of the upper component 112. However, in other embodiments the width 146 varies along the length and/or height of the engagement structure 132. For example, in some embodiments the width 146 is narrowed towards at least one end and/or the upper portion of the engagement structure 132 compared to the other portions of the structure. In some embodiments, the width 146 is narrowed such that the engagement structure 132 is capable of cutting into the bone structure to further facilitate engagement of the prosthetic device 110 with the adjacent vertebrae V_{U}, V_{L}. Further, in some embodiments the engagement structure 132 is sharp enough that it is capable of being inserted without a preformed opening in the adjacent vertebrae. In some embodiments, the width 146 varies such that the surfaces 142 and 144 are non-planar. Also, the width 146 of the engagement structure 132 is different from the width 138 of the engagement structure 124 in some embodiments. In general, the width 146 of the engagement structure 132 is within a range of .5 mm to 10 mm, but may be smaller or greater in some embodiments.

The engagement structure 132 also includes a pair of openings 148 extending therethrough between the opposing surfaces 142, 144 to facilitate bone in-growth to enhance fixation to the adjacent vertebra. However, it should be understood that any number of openings may be defined through the engagement structure 132, including a single opening or three or more openings. It should also be understood that the openings 148 need not necessarily extend entirely through the engagement structure 132, but may alternatively extend partially therethrough. It should further be understood that the engagement structure 132 need not necessarily define any openings extending either partially or entirely therethrough. Additionally, although the openings 148 are illustrated as having a circular configuration, it should be understood that other shapes, sizes, and configurations of the openings are also contemplated.

As shown in Figure 9, when the prosthetic device 110 is in a neutral position the engagement structure 132 extends in a direction substantially parallel to the engagement structure 124 of the upper component 112, but offset along the longitudinal axis. In other embodiments, the engagement structure 132 is at an angle with respect to the engagement structure 124 when the prosthetic device 110 is in a neutral position. In some embodiments, the engagement structure 132 is substantially perpendicular to the engagement structure 124. In yet other embodiments, the engagement structure 132 is substantially aligned with the engagement structure 124.

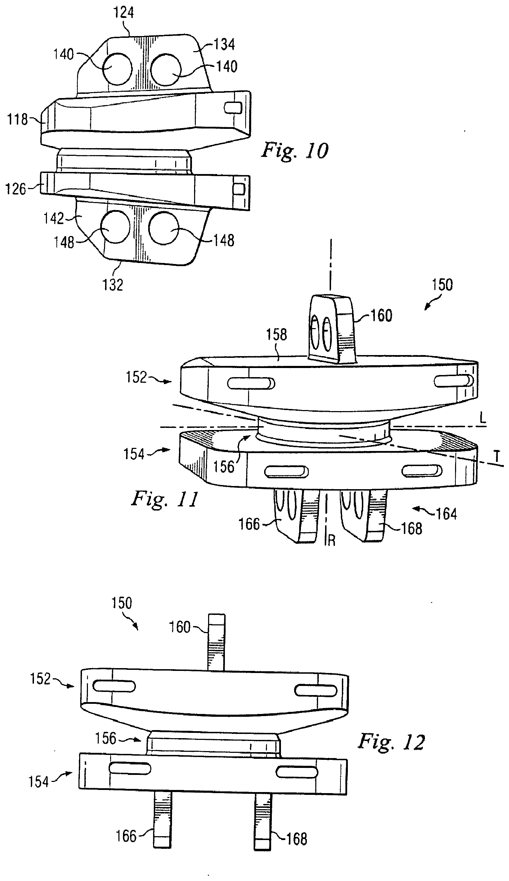

Referring to Figures 11-13, shown therein is another embodiment of a prosthetic device 150 according to another aspect of the present disclosure. The prosthetic device 150 is an alternative to the prosthetic devices 20, 110 described above. In particular, the prosthetic device 150 is substantially similar in some aspects to the prosthetic device 110 described above. Therefore, only portions of the prosthetic device 150 will be described in detail.

The prosthetic device 150 includes an upper component 152, a lower component 154, and an articulating central portion 156. The upper component 152 includes an engagement surface 158 having an engagement structure 160 extending therefrom. The engagement structure 160 is substantially similar to the engagement structures 124 and 132 described above. In the current embodiment, the engagement structure 160 is substantially transverse to the engagement surface 158 and longitudinal axis L of the prosthetic device. The engagement structure 160 is positioned centrally along the longitudinal axis L, as best seen in Figure 12.

Referring again to Figures 11-13, the lower component 154 includes an engagement surface 162 having an engagement structure 164 extending therefrom. The engagement structure 164 includes a pair of flange members 166 and 168, each of which is substantially similar to the engagement structure 160. In the current embodiment, the flange members 166 and 168 are substantially transverse to the engagement surface 162 and the longitudinal axis L of the prosthetic device 150. The flange members 166 and 168 are offset symmetrically from a center point of the longitudinal axis L, as best seen in Figure 12. Thus, the flange members 166 and 168 are equally spaced along the longitudinal axis L from the engagement structure 160. In other embodiments, the engagement structures 160, 164 each may have more or less flange members and be positioned in other orientations.

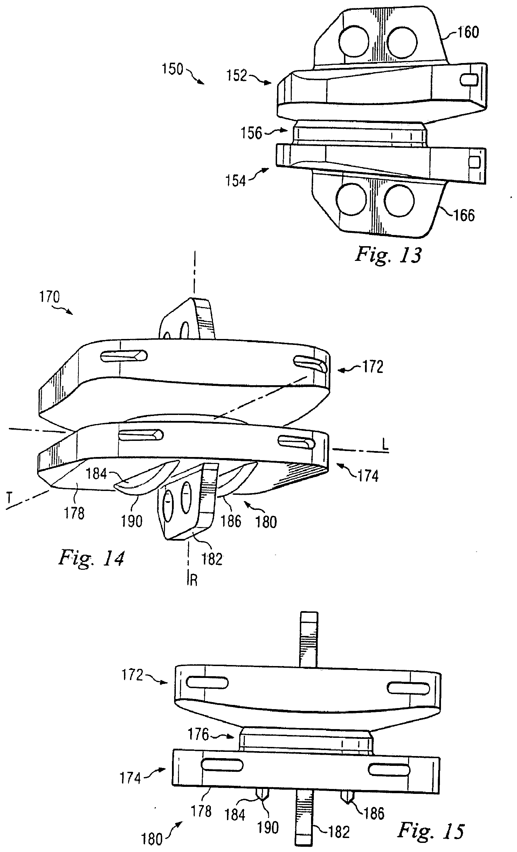

Referring to Figures 14-17, shown therein is another embodiment of a prosthetic device 170 according to another aspect of the present disclosure. The prosthetic device 170 is an alternative to the prosthetic devices 20, 110, 150 described above. In particular, the prosthetic device 170 is substantially similar in some aspects to the prosthetic devices 110 and 150 described above. Therefore, only portions of the prosthetic device 170 will be described in detail.

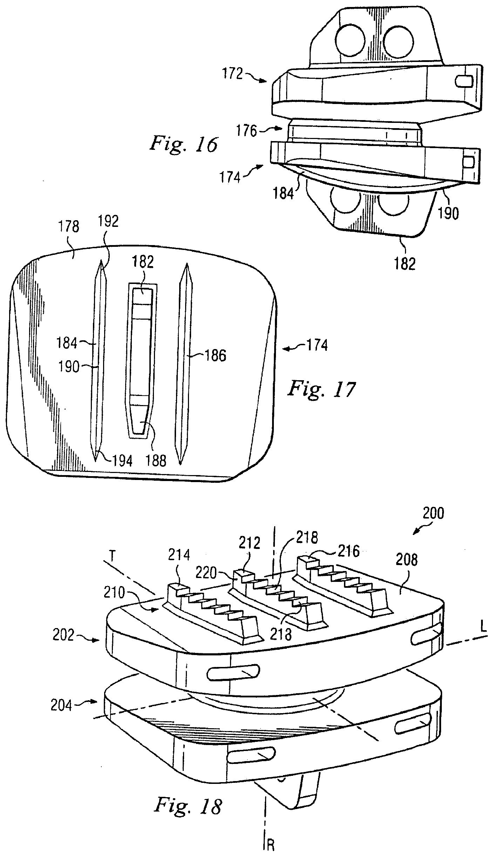

The prosthetic device 170 includes an upper component 172, a lower component 174, and an articulating central portion 176. As shown, the upper component 172 is substantially similar to the upper component 152 described above and, therefore, will not be described in detail. The lower component 174 includes an engagement surface 178 having an engagement structure 180 extending therefrom. The engagement structure 180 includes a major flange member 182 and a pair of minor flange members 184 and 186. The flange member 182 is substantially similar to the engagement structures 124, 132, 160, and 166 described above. However, the flange member 182 includes a tapered portion 188, as best seen in Figure 17. The tapered portion 188 serves as the leading portion of the flange member 182 during insertion to help facilitate insertion of the flange member into a prepared opening in the adjacent vertebra. The flange member 182 is substantially transverse to the engagement surface 178 and longitudinal axis L of the prosthetic device 170. The flange member 182 is positioned centrally along the longitudinal axis L, as best seen in Figures 15 and 17.

Referring again to Figures 14-17, the flange member 184 and the flange member 186 are substantially similar structurally. Therefore, only the flange member 184 will be described in detail here. In general, the flange member 184 is adapted for cutting into the adjacent bone structure, such as an adjacent vertebra, to further facilitate engagement between the prosthetic device 170 and the bone structure. Thus, the flange member 184 is capable of being inserted into the bone structure without the need for preparing an opening in the bone structure to receive the flange member. However, in some embodiments the flange member 184 is inserted into a prepared opening in the bone structure. The flange member 184 includes a sharpened edge 190 that extends substantially along its length. The edge 190 has substantially arcuate shape, as best seen in Figures 14 and 16. The flange member 184 also includes tapered end portions 192 and 194, as shown in Figure 17. In some embodiments, the tapered end portions 192 and 194 facilitate initial engagement with and/or the initial cutting of the bone structure.

The flange members 184 and 186 are substantially transverse to the engagement surface 178 and the longitudinal axis L of the prosthetic device 170. The flange members 184 and 186 are substantially parallel to one another. The flange members 184 and 186 are also substantially parallel to the flange member 182. The flange member 184 and 186 are symmetrically offset from the flange member 182, as shown in Figures 15 and 17. In other embodiments, the flange members 184, 186 are non-parallel to one another and/or non-parallel to the flange member 182. In some embodiments, the flange members 184, 186 are non-symmetrically offset with respect to the flange member 182. In some embodiments, the engagement structure 180 includes more or less major flange members and/or more or less minor flange members in various orientations.

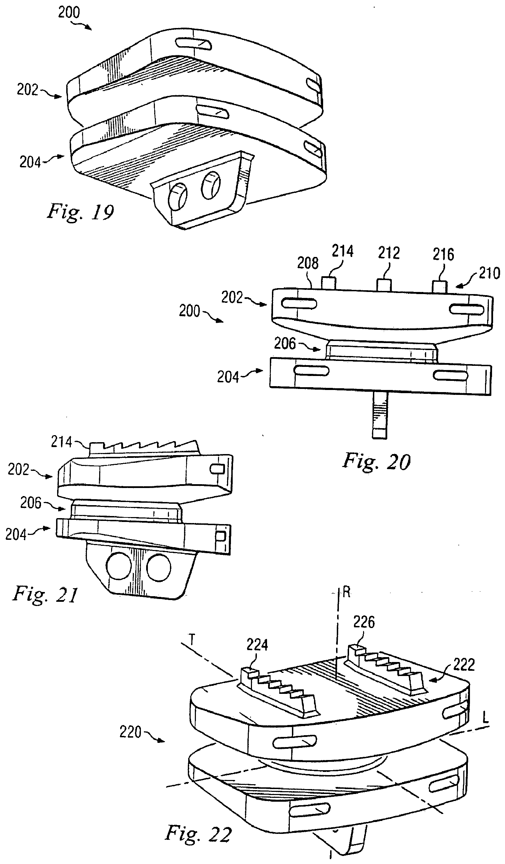

Referring to Figures 18-21, shown therein is another embodiment of a prosthetic device 200 according to another aspect of the present disclosure. The prosthetic device 200 is an alternative to the prosthetic devices 20, 110, 150, and 170 described above. In particular, the prosthetic device 200 is substantially similar in some aspects to the prosthetic devices described above. Therefore, only portions of the prosthetic device 200 will be described in detail. The prosthetic device 200 includes an upper component 202, a lower component 204 and an articulating central portion 206. As shown in Figure 19, the features of the lower component 204 are substantially similar in many respects to the upper components 152 and 172 described above and, therefore, will not be described in detail here.

Referring to Figures 18-21, the upper component 202 includes an engagement surface 208 having an engagement structure 210 extending therefrom. In the current embodiment, the engagement structure 210 includes three flange members 212, 214, and 216. The flange members 212, 214, 216 are substantially similar structurally. Therefore, only the flange member 212 will be described in detail here. The flange member 212 is adapted to be inserted into a prepared opening in the adjacent bone structure, such as an adjacent vertebra. In some embodiments, the flange member 212 is inserted into the bone structure without preparing an opening in the bone structure to receive the flange member. The flange member 212 includes a plurality of teeth or projections 218 for engaging the bone structure. In the current embodiment, the projections 218 are positioned substantially on the upper portion of the flange member 212 and comprise a series of alternating sharpened edges and recesses. The flange member 212 also includes a tapered portion 220, as best seen in Figure 18. The tapered portion 220 serves as the leading portion of the flange member 212 during insertion to help facilitate insertion of the flange member into a prepared opening in the adjacent vertebra.

Referring to Figure 20, the flange member 212 has a height that is substantially equal to its width. In general the height and/or width of the engagement structure 212 is within a range of .5 mm to 10 mm, but each may be smaller or greater in some embodiments. Further, in some embodiments the height and width of the flange member 212 are different. As shown, the height of the flange members 212 is substantially less than that of the engagement structure of the lower component 204. In other embodiments, however, the height of the flange member 212 is substantially equal to or greater than the height of the engagement structure of the lower component 204. Further, the width of the flange members 212 is substantially equal to the width of the engagement structure of the lower component 204. In other embodiments, however, the width of the flange member 212 is greater or lesser than the height of the engagement structure of the lower component 204.

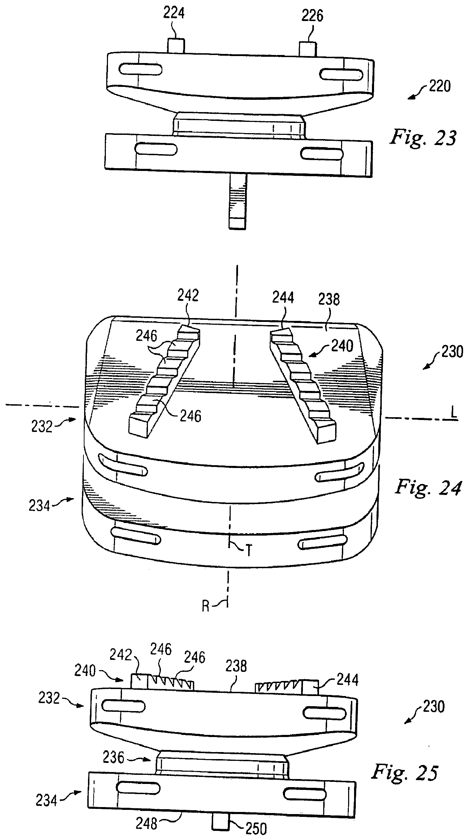

Referring to Figures 18-21 again, the flange members 212, 214, and 216 are substantially transverse to the engagement surface 208 and longitudinal axis L of the prosthetic device 200. In the current embodiment, the flange members 212, 214, and 216 are substantially parallel to one another. Referring more specifically to Figure 20, the flange member 212 is positioned centrally along the longitudinal axis L. The flange members 214 and 216 are symmetrically offset from the flange member 212. In other embodiments, the flange members 214 and 216 are non-parallel to one another and/or non-parallel to the flange member 212. In some embodiments, the flange members 214, 216 are non-symmetrically offset with respect to the flange member 182. In some embodiments, the engagement structure 210 includes more or less flange members in various orientations. Referring to Figures 22 and 23, for example, shown therein is a prosthetic device 220 substantially similar the prosthetic device 200, except that the flange member 212 of the engagement structure 210 has been removed. Thus, the engagement structure 222 includes only two flange members 224 and 226.

Referring to Figures 24-26, shown therein is another embodiment of a prosthetic device 230 according to another aspect of the present disclosure. The prosthetic device 230 is an alternative to the prosthetic devices 20, 110, 150, 170, 200, and 220 described above. The prosthetic device 230 is substantially similar in some aspects to the prosthetic devices described above. Therefore, only portions of the prosthetic device 230 will be described in detail.

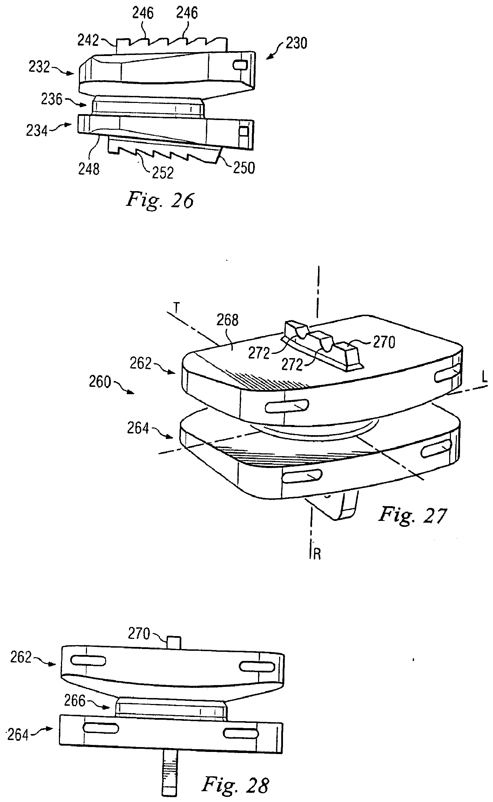

The prosthetic device 230 includes an upper component 232, a lower component 234 and an articulating portion 236. The upper component 232 includes an engagement surface 238 having an engagement structure 240 extending therefrom. In the current embodiment, the engagement structure 240 includes a pair of flange members 242 and 244. The flange members 242 and 244 are substantially similar structurally. Therefore, only the flange member 242 will be described in detail here. The flange member 242 is adapted to be inserted into a prepared opening in the adjacent bone structure, such as an adjacent vertebra. In some embodiments, the flange member 242 is inserted into the bone structure without preparing an opening in the bone structure to receive the flange member. The flange member 242 includes a plurality of teeth or projections 246 for engaging the bone structure. In the current embodiment, the projections 246 are positioned substantially on the upper portion of the flange member 242 and comprise a series of alternating projections and recesses. Further, in the current embodiment the edges of the projections 246 run substantially parallel to the longitudinal axis L of the prosthetic device 230, as best seen in Figures 24 and 26. Thus, as shown the edges of the projections 246 are at an oblique angle to a longitudinal axis extending substantially along the length of the flange member 242. In other embodiments, the edges of the projections are substantially perpendicular to the longitudinal axis extending substantially along the length of the flange member 242. Thus, in some embodiments, the edges of the teeth 246 do not run substantially parallel to the longitudinal axis L of the prosthetic device 230.

Referring to Figure 25, the flange member 242 has a height that is substantially equal to its width. In general the height and/or width of the engagement structure 242 is within a range of .5 mm to 10 mm, but each may be smaller or greater in some embodiments. Further, in some embodiments the height and width of the flange member 242 are different. As shown, the height of the flange members 242 is equal to that of the flange member 244. In other embodiments, however, the height of the flange member 242 is greater or lesser than the height of the flange member 244. Similarly, the width of the flange members 242 is substantially equal to the width of the flange member 244, but in other embodiments the height of the flange member 242 is greater or lesser than the width of the flange member 244.

Referring to Figures 24 and 25, the flange members 242 and 244 are substantially transverse to the engagement surface 238. The flange members 242 and 244 extend from the engagement surface 238 at an oblique angle to the longitudinal axis L of the prosthetic device 230. Further, in the current embodiment the flange members 242 and 244 are at an oblique angle to one another as well. However, in the flange members 242 and 244 are symmetrically offset from transverse axis T. In other embodiments, the flange members 242 and 244 have other orientations to one another. For example, flange members 242 and 244 are substantially parallel in some embodiments and substantially transverse to one another in other embodiments. Further, in some embodiments the flange members 242 and 244 are non-symmetrically offset with respect to the transverse axis T. In some embodiments, the engagement structure 240 includes more or less flange members in various orientations.

The lower component 204 includes an engagement surface 248 having an engagement structure 250 extending therefrom. In the current embodiment, the engagement structure 250 comprises a single flange member. The engagement structure 250 is adapted to be inserted into a prepared opening in the adjacent bone structure, such as an adjacent vertebra. In some embodiments, the engagement structure 250 is inserted into the bone structure without preparing an opening in the bone structure to receive the engagement structure. The engagement structure 250 includes a plurality of teeth or projections 252 for engaging the bone structure. In the current embodiment, the projections 252 are positioned substantially on the upper portion of the engagement structure 250 and comprise a series of alternating sharpened edges and recesses.

Referring to Figures 25 and 26, the engagement structure 250 is substantially transverse to the engagement surface 248 and the longitudinal axis L of the prosthetic device 230. Referring more specifically to Figure 25, the engagement structure 250 is positioned centrally along the longitudinal axis L. The engagement structure 250 has a height that is substantially equal to its width. In general the height and/or width of the engagement structure 250 is within a range of .5 mm to 10 mm, but each may be smaller or greater in some embodiments. Further, in some embodiments the height and width of the engagement structure 250 are different. As shown, the height of the engagement structure 250 is substantially equal to that of the flange members 242 and 244 of the engagement structure 240 of the upper component 232. In other embodiments, however, the height of the engagement structure 250 is greater and/or lesser than the heights of the engagement structure 240 of the upper component 232. Further, the width of the engagement structure 250 is substantially equal to the width of the the flange members 242 and 244 of the engagement structure 232 of the upper component 204. In other embodiments, however, the width of the engagement structure 250 is greater and/or lesser than the widths of the engagement structure of the upper component 232.

Referring to Figures 27-29, shown therein is another embodiment of a prosthetic device 260 according to another aspect of the present disclosure. The prosthetic device 260 is an alternative to the prosthetic devices 20, 110, 150, 170, 200, 220, and 230 described above. The prosthetic device 260 is substantially similar in some aspects to the prosthetic devices described above. Therefore, only portions of the prosthetic device 260 will be described in detail. The prosthetic device 260 includes an upper component 262, a lower component 264 and an articulating central portion 266. The features of the lower component 264 are substantially similar in many respects to lower component 204 and the upper components 152 and 172 described above and, therefore, will not be described in detail here.

The upper component 262 includes an engagement surface 268 having an engagement flange 270 extending therefrom. In the current embodiment, the engagement flange 270 is adapted to be inserted into a prepared opening in the adjacent bone structure, such as an adjacent vertebra. In some embodiments, the flange 270 is inserted into the bone structure without preparing an opening in the bone structure to receive the flange member. The flange 270 includes a pair of recesses 272. In the current embodiment, the recesses 272 are positioned substantially on the upper portion of the flange 270 and are substantially arcuate. In other embodiments, the recesses 272 have other shapes, configurations, and locations along the flange 270. Further, in some embodiments the engagement flange 270 includes more or less recesses 272. The flange 270 also includes a tapered portion 274, as best seen in Figure 27. The tapered portion 274 serves as the leading portion of the flange 270 during insertion to help facilitate insertion of the flange member into a prepared opening in the adjacent vertebra.

Referring more specifically to Figure 28, the flange 270 has a height that is substantially equal to its width. In general the height and/or width of the engagement flange 270 is within a range of .5 mm to 10 mm, but each may be smaller or greater in some embodiments. Further, in some embodiments the height and width of the flange 270 are different. As shown, the height of the flange members 270 is substantially less than that of the engagement structure of the lower component 264. In other embodiments, however, the height of the flange 270 is substantially equal to or greater than the height of the engagement structure of the lower component 264. Further, the width of the flange 270 is substantially equal to the width of the engagement structure of the lower component 264. In other embodiments, however, the height of the flange 270 is greater or lesser than the height of the engagement structure of the lower component 264.

Referring to Figures 27-29 again, the engagement flange 270 is substantially transverse to the engagement surface 268 and the longitudinal axis L of the prosthetic device 260. The flange 270 is positioned centrally along the longitudinal axis L. In the current embodiment, the flange 270 is in substantial alignment with the engagement structure of the lower component 264. In other embodiments, however, the flange 270 is offset with respect to the engagement structure of the lower component 264. Further, in other embodiments, the flange 270 is located in other positions and orientations in other embodiments, including off-center and/or at oblique angles relative to the axes of the prosthetic device 260.

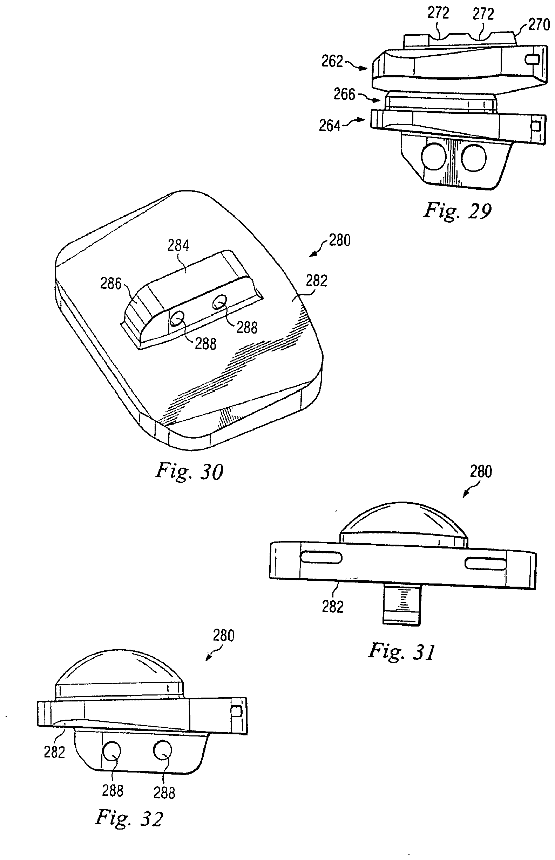

Referring to Figures 30-33, shown therein is a component 280 of a prosthetic device according to another aspect of the present disclosure. The lower component 280 is an alternative to the upper and lower components of the prosthetic devices described above. In particular, the component 280 is substantially similar in some aspects to the components 24, 80, 114, 152, 154, 172, 174, 204, 234, and 264 described above. Therefore, only portions of the component will be described in detail.

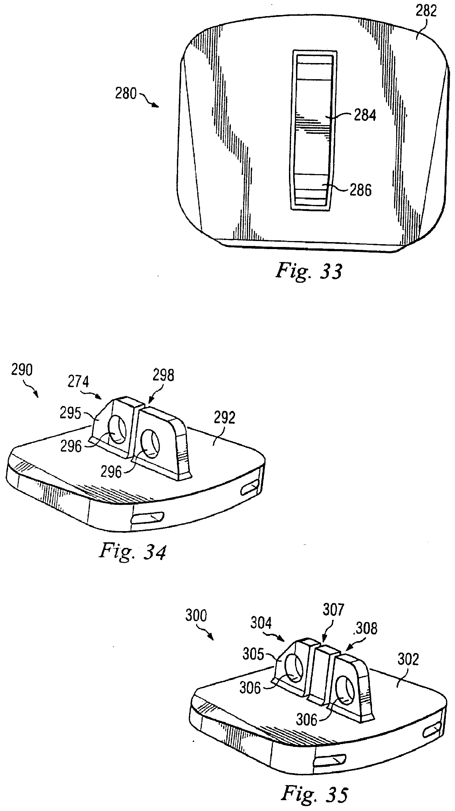

The component 280 includes an engagement surface 282 having an engagement structure 284 extending therefrom. The engagement structure 284 is configured for disposition within a preformed opening in the adjacent vertebral endplate. The engagement structure 284 is substantially similar, in some aspects, to the engagement structures 124, 132, 160, 166, and 182 described above. As shown, however, the engagement structure 284 has a greater height and a greater width than similar engagement structures above. Similar to the previous structures, the engagement structure 284 is substantially transverse to the engagement surface 282 and the longitudinal axis L of the component 280. Further, the engagement structure 284 is positioned centrally along the longitudinal axis L, as best seen in Figures 31 and 33. Also, the engagement structure 284 includes a tapered portion 286, as best seen in Figure 33. The tapered portion 286 serves as the leading portion of the engagement structure 284 during insertion to help facilitate insertion of the structure into a prepared opening in the adjacent vertebra. The engagement structure 284 also includes a pair of openings 288 extending therethrough.

Referring to Figure 34, shown therein is a component 290 of a prosthetic device according to another aspect of the present disclosure. The component 290 is an alternative to the upper and lower components of the prosthetic devices described above. In particular, the component 290 is substantially similar in some aspects to the components 24, 80, 114, 152, 154, 172, 174, 204, 234, 264, and 280 described above. Therefore, only portions of the component 290 will be described in detail.

The component 290 includes an engagement surface 292 having an engagement structure 294 extending therefrom. The engagement structure 294 is configured for disposition within a preformed opening in the adjacent vertebral endplate. The engagement structure 294 is substantially similar, in some aspects, to the engagement structures 124, 132, 160, 166, 182, and 284 described above. Similar to the previous structures, the engagement structure 294 is substantially transverse to the engagement surface 292 and the longitudinal axis L of the component 290. Further, the engagement structure 294 is positioned centrally along the longitudinal axis L. Also, the engagement structure 294 includes a tapered portion 295. The tapered portion 295 serves as the leading portion of the engagement structure 294 during insertion to help facilitate insertion of the structure into a prepared opening in the adjacent vertebra. Also similar to the previous structures, the engagement structure 294 includes a pair of openings 296 extending therethrough.

As shown in Figure 34, however, the engagement structure 294 also includes an elongated slot 298. In the present embodiment, the slot 298 extends completely through the engagement structure 294 so as to bifurcate the structure into two parts. In other embodiments, the slot extends only partially across the width and/or height of the engagement structure 294. In the present embodiment, the elongated slot 298 is approximately centrally located along the length of the engagement structure 294. In other embodiments, the slot is positioned closer to one end of the engagement structure 294. Further, in the current embodiment the slot 298 is substantially rectangular. In other embodiments, the slot 298 has other geometrical and non-geometrical shapes.

Referring to Figure 35, shown therein is a component 300 of a prosthetic device according to another aspect of the present disclosure. The component 300 is an alternative to the upper and lower components of the prosthetic devices described above. In particular, the component 300 is substantially similar in some aspects to the components 24, 80, 114, 152, 154, 172, 174, 204, 234, 264, and 280 described above. Therefore, only portions of the component 300 will be described in detail.

The component 300 includes an engagement surface 302 having an engagement structure 304 extending therefrom. The engagement structure 304 is configured for disposition within a preformed opening in the adjacent vertebral endplate. The engagement structure 304 is substantially similar, in some aspects, to the engagement structures 124, 132, 160, 166, 182, 284, and 294 described above. Similar to the previous structures, the engagement structure 304 is substantially transverse to the engagement surface 302 and the longitudinal axis L of the component 300. Further, the engagement structure 304 is positioned centrally along the longitudinal axis L. Also, the engagement structure 304 includes a tapered portion 305. The tapered portion 305 serves as the leading portion of the engagement structure 304 during insertion to help facilitate insertion of the structure into a prepared opening in the adjacent vertebra. Also similar to the previous structures, the engagement structure 304 includes a pair of openings 306 extending therethrough.

As shown in Figure 35, however, the engagement structure 304 also includes a pair of slots 307, 308 extending therethrough. In the current embodiment, the slots 307, 308 are substantially similar to slot 298 described above. In the present embodiment, the slots 307, 308 extend completely through the engagement structure 304 so as to divide the structure into three parts. In the present embodiment, the slots 307, 308 are approximately equally spaced from or symmetrical about a center point along the length of the engagement structure 304. Further, in the current embodiment the slots 307, 308 are substantially rectangular. In other embodiments, however, one or both of the slots extend only partially across the width and/or height of the engagement structure 304; one or both of the slots are positioned closer to one end of the engagement structure; the slots are positioned non-symmetrically about the center point of the length of the engagement structure; and/or one or both of the slots have other geometrical and non-geometrical shapes.

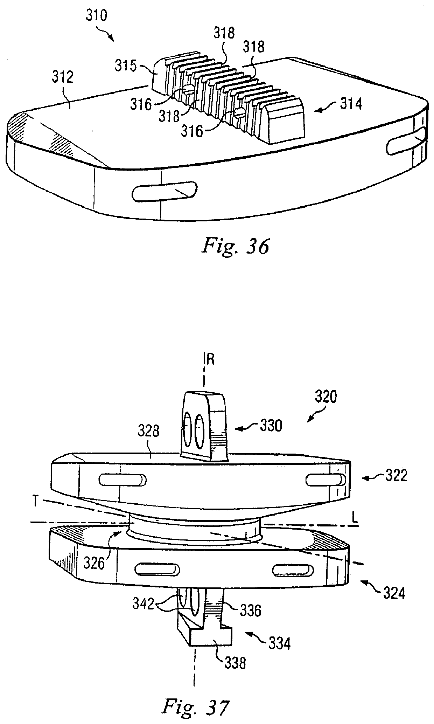

Referring to Figure 36, shown therein is a component 310 of a prosthetic device according to another aspect of the present disclosure. The component 310 is an alternative to the upper and lower components of the prosthetic devices described above. In particular, the component 310 is substantially similar in some aspects to the components 24, 80, 114, 152, 154, 172, 174, 204, 234, 264, 280, 290, and 300 described above. Therefore, only portions of the component 300 will be described in detail.

The component 310 includes an engagement surface 312 having an engagement structure 314 extending therefrom. The engagement structure 314 is substantially similar, in some aspects, to the engagement structures 124, 132, 160, 166, 182, 284, 294, and 304 described above. Similar to the previous structures, the engagement structure 314 is configured for disposition within a preformed opening in the adjacent vertebral endplate. Also, the engagement structure 314 is substantially transverse to the engagement surface 312 and the longitudinal axis L of the component 310. Further, the engagement structure 314 is positioned centrally along the longitudinal axis L. Also, the engagement structure 314 includes a tapered portion 315. The tapered portion 315 serves as the leading portion of the engagement structure 314 during insertion to help facilitate insertion of the structure into a prepared opening in the adjacent vertebra. Also similar to the previous structures, the engagement structure 314 includes a pair of openings 316 extending therethrough.

The engagement structure 314 also includes a plurality of engagement ridges 318 extending from its sides and/or top. In the present embodiment, the ridges 318 comprise a series of alternating projections and recesses. The ridges 318 provide additional engagement with the adjacent bone structure, such as a vertebral endplate. In the current embodiment, the projections include sharp or pointed edges to facilitate engagement with the adjacent bone structure. In other embodiments, the ridges 318 include other structural features to facilitate engagement.

Referring now to Figures 37-39, shown therein is another embodiment of a prosthetic device 320 according to another aspect of the present disclosure. The prosthetic device 320 is an alternative to the prosthetic devices described above. In particular, the prosthetic device 320 is substantially similar in some aspects to the prosthetic devices described above. Therefore, only portions of the prosthetic device 320 will be described in detail.

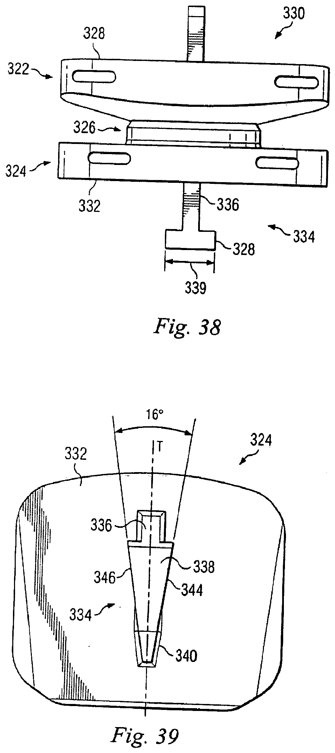

The prosthetic device 320 includes an upper component 322, a lower component 324, and an articulating central portion 326. The upper component 322 includes an engagement surface 328 having an engagement structure 330 extending therefrom. The engagement structure 330 is substantially similar to the engagement structures 124, 132, 160, and 182 described above. In the current embodiment, the engagement structure 330 is substantially transverse to the engagement surface 328 and longitudinal axis L of the prosthetic device 320. The engagement structure 330 is positioned centrally along the longitudinal axis L, as best seen in Figure 38.

Referring again to Figures 37-39, the lower component 324 includes an engagement surface 332 having an engagement structure 334 extending therefrom. The engagement structure 334 comprises flange member 336 and a wing portion 338. In the current embodiment, the flange member 336 and the wing portion 338 are integrally formed. In some aspects, the flange member 336 is substantially similar to the engagement structures 124, 132, 160, 182, and 330 described above. In the current embodiment, the flange member 336 is substantially transverse to the engagement surface 332 and the longitudinal axis L of the prosthetic device 320. The flange member 336 is also positioned centrally along the longitudinal axis L, as best seen in Figure 38. The flange member 336 also includes a tapered portion 340. The tapered portion 340 serves as the leading portion of the engagement structure 334 during insertion to help facilitate insertion of the structure into a prepared opening in the adjacent vertebra. Further, as shown in Figure 37, the flange member 336 includes a pair of openings 342 extending therethrough.

As shown Figures 37-39, the wing portion 338 bounds the flange member 336 at an end opposite the engagement surface 332. The wing portion 338 extends substantially transverse to the flange member 336. As shown the wing portion 338 has a width 339 that is greater than the width of the flange member 336 such that wing portion extends beyond the flange member at some points. In this regard, the flange member 336 and the wing portion 338 form a t-shaped cross-section at one end of the engagement structure 334. Thus, in the current embodiment the wing portion 338 is substantially parallel to the engagement surface 332. In other embodiments, the wing portion 338 is at least partially at an oblique angle with respect to the flange member 336 and/or the engagement surface 332. In the current embodiment, the wing portion 338 can engage the adjacent bone structure to further prevent unwanted translation of the lower component 324 along the rotational axis R after insertion of the prosthetic device 320.

The wing portion 338 tapers along its length. In the current embodiment, the wing portion 338 tapers at the same angle or degree as the tapered portion 340 of the flange member 336. Thus, as shown in Figure 39, outer edges 344, 346 of the wing portion 338 are substantially aligned with the outer edges of the tapered portion 340. In other embodiments, however, the wing portion 338 tapers at a different angle(s) or degree(s) compared to the tapered portion 340 of the flange member. In the current embodiment, the wing portion 338 is tapered such that its outer edges 344, 346 extend from the transverse axis T of the device 320 at an angle of approximately 8°. In some aspects, the outer edges 344, 346 of the wing portion 338 extend from the transverse axis T at an angle between 0° and 90°, and more particularly between 2° and 45°. In some aspects, the outer edges 344, 346 are substantially perpendicular to the transverse axis T. In other aspects, the outer edges 344, 346 are substantially parallel to the transverse axis T. In yet other aspects, the outer edges 344, 346 are arcuate. In some aspects, the wing portion 338 does not taper along its length.

The components of the various prosthetic devices described above may be formed of any suitable biocompatible material including metals such as cobalt-chromium alloys, titanium alloys, nickel titanium alloys, or stainless steel alloys. Ceramic materials such as aluminum oxide or alumina, zirconium oxide or zirconia, compact of particulate diamond, or pyrolytic carbon may also be suitable. Polymer materials may also be used, including any member of the polyaryletherketone (PAEK) family such as polyetheretherketone (PEEK), carbon-reinforced PEEK, or polyetherketoneketone (PEKK); polysulfone; polyetherimide; polyimide; ultra-high molecular weight polyethylene (UHMWPE); or cross-linked UHMWPE. Further, the components may each be formed of different materials, permitting metal on metal, metal on ceramic, metal on polymer, ceramic on ceramic, ceramic on polymer, or polymer on polymer constructions. Further, the articulating surfaces and the articulating portions described above may be treated to limit friction and resulting wear debris caused by rotational movement. Further, the surfaces of the prosthetic devices that are positioned in direct contact with the bone structure, such as a vertebra, may be coated with a bone-growth promoting substance that would occur to one of ordinary skill in the art. Additionally, the surface of the prosthetic devices that are positioned in direct contact with vertebral bone may be roughened to further enhance bone in-growth. Such surface roughening may be accomplished by way of acid etching, knurling, application of a bead coating, or other methods of roughening that would occur to one of ordinary skill in the art. Further, surface roughening may be used in combination with bone-growth promoting substances.

Other modifications of the present disclosure would be apparent to one skilled in the art. Accordingly, all such modifications and alternatives are intended to be included within the scope of the invention as defined in the following claims. Those skilled in the art should also realize that such modifications and equivalent constructions or methods do not depart from the spirit and scope of the present disclosure, and that they may make various changes, substitutions, and alterations herein. It is understood that all spatial references, such as "horizontal," "vertical," "top," "upper," "lower," "bottom," "left," and "right," are for illustrative purposes only and can be varied within the scope of the disclosure. In the claims, means-plus-function clauses are intended to cover the structures described herein as performing the recited function and not only structural equivalents, but also equivalent structures.

## Claims

1. An intervertebral articulating prosthetic device (20) having a longitudinal axis (L), a transverse axis (T) and a vertical axis (R), and comprising:
an upper component (22), a lower component (24), and an articulating central portion (26) between the upper and lower components (22, 24), by means of which the upper and lower components (22, 24) are moveably engaged with each other, the upper component (22) having a first engagement surface (32) and a first engagement structure (34) having a pair of opposite surfaces (48, 50), separated by a width (52), and extending from the first engagement surface (32) at a first oblique angle between the first engagement structure (34) and the first engagement surface (32), and comprising a single flange member or keel extending substantially across the first engagement surface (32) in a direction substantially along the transverse axis (T), the first engagement structure (34), along the longitudinal axis (L), being approximately centrally located along the corresponding longitudinal dimension of the device (20) and being configured for disposition within a preformed opening in an adjacent vertebral endplate; and
the lower component (24) having a second engagement surface (40) and a second engagement structure (42) having a pair of opposite surfaces (60, 62) separated by a width and extending from the second engagement surface (40) at a second oblique angle about the transverse axis (T) and comprising a single flange member or keel extending substantially across the second engagement surface (40) in a direction substantially transverse to the longitudinal axis (L) and extending from the second engagement surface (40) at a second oblique angle about the transverse axis (T), the second engagement structure (42) being approximately centrally located along the longitudinal axis (L) and being configured for disposition within a preformed opening in an adjacent vertebral endplate.

2. The prosthetic device (20) of claim 1 wherein the first engagement structure (34) is substantially parallel to the second engagement structure (42) when the first engagement surface (32) is substantially parallel to the second engagement surface (40).

3. The prosthetic device (20) of claim 2 wherein the first engagement structure (34) is substantially similar to the second engagement structure (42).

4. The prosthetic device (20) of claim 3 wherein the pair of opposite surfaces (48, 50; 60, 62) of each of the first and second engagement structures (34; 42) are substantially parallel and extend along a majority of the length of the flange member.

5. The prosthetic device (20) of claim 4 wherein the width (52; 64) of the respective flange member (34; 42) is substantially constant along the length of the flange member (34; 42).

6. The prosthetic device (20) of claim 4 wherein the width (52; 64) of the respective flange member (34; 42) is tapered at at least one end of the flange member (34; 42).

7. The prosthetic device (20) of claim 6 wherein the first and second engagement structures (34; 42) further comprise at least one opening (54) extending through the corresponding flange member between the corresponding parallel surfaces (48, 50; 60, 62).

8. An intervertebral articulating prosthetic device for disposition within a space between a pair of vertebrae, having a longitudinal axis (L), a transverse axis (T) and a vertical axis (R), the device comprising:
a first component (80) for engaging with the first vertebra, a second component for engaging with the second vertebra, and an articulating central portion between the first and second components, by means of which the upper and lower components (22, 24) are moveably engaged with each other, the first component (80) having: a first engagement surface (82) which is substantially planar, and a first engagement structure (84) extending from the first engagement surface (82), the first engagement structure (84) comprising a first pair of flange members (86, 88) and, along the longitudinal axis (L), being approximately centrally located along the corresponding longitudinal dimension of the device, each flange member (86, 88) having a pair of opposing surfaces (90,92; 100,102), which are separated by a width (94, 104), and extending in a direction substantially along the transverse axis (T) and extending from the first engagement surface (82) at an oblique angle between the respective flange member (86, 88) and the first engagement surface (82) such that the flange members (86,88) are substantially perpendicular to one another, and each of the flange members (86, 88) extends substantially across the first engagement surface (82) and the flange members (86, 88) are configured for disposition within preformed openings in an adjacent vertebral endplate.

9. The prosthetic device (80) of claim 8 wherein the first pair of flange members (86, 88) are substantially symmetrical to one another about a plane passing through a center point of the first engagement surface (82) and extending substantially transverse to the first engagement surface (82).

10. The prosthetic device (80) of claim 9 wherein the plane is also substantially transverse to the length of the first engagement surface (82).

11. The prosthetic device (80) of claim 10 wherein each of the first pair of flange members (86, 88) has a substantially constant width along its length.

12. The prosthetic device (80) of claim 11 wherein each of the first pair of flange members (86, 88) has a substantially constant height along its length.

13. The prosthetic device (80) of claim 8 wherein the second component comprises
a second engagement surface, and
a second engagement structure extending from the second engagement surface, the second engagement structure comprising a second pair of flange members, each flange member extending from the second engagement surface at an oblique angle such that the flange members are substantially perpendicular to one another.

14. The prosthetic device (80) of claim 13 wherein at least one of the second pair of flange members is substantially parallel to one of the first pair of flange members when the second engagement surface is substantially parallel to the first engagement surface.

15. The prosthetic device (80) of claim 14 wherein each of the second pair of flange members is substantially parallel to one of the first pair of flange members when the second engagement surface is substantially parallel to the first engagement surface.

## Patentansprüche

1. Eine Zwischenwirbel-Gelenk-Prothesenvorrichtung (20), die eine Längsachse (L), eine Querachse (T) und eine Vertikalachse (R) hat und aufweist:
eine obere Komponente (22), eine untere Komponente (24) und einen Gelenk-Zentralabschnitt (26) zwischen der oberen und der unteren Komponente (22, 24), mittels dessen die obere und die untere Komponente (22, 24) in bewegbarem Eingriff miteinander sind, wobei die obere Komponente (22) eine erste Eingriffsfläche (32) und eine erste Eingriffsstruktur (34) hat, die ein Paar von entgegengesetzten Flächen (48, 50) hat, die durch eine Breite (52) getrennt sind, und die sich von der ersten Eingriffsfläche (32) in einem ersten schiefen Winkel zwischen der ersten Eingriffsstruktur (34) und der ersten Eingriffsfläche (32) erstreckt und ein einziges Flanschelement oder Kiel aufweist, das/der sich im Wesentlichen quer über die erste Eingriffsfläche (32) in einer Richtung im Wesentlichen entlang der Querachse (T) erstreckt, wobei die erste Eingriffsstruktur (34) entlang der Längsachse (L) annähernd zentral entlang der korrespondierenden Längsabmessung der Vorrichtung (20) angeordnet ist und für eine Anordnung innerhalb einer vorgeformten Öffnung in einer benachbarten Wirbelendplatte konfiguriert ist, und wobei die untere Komponente (24) eine zweite Eingriffsfläche (40) und eine zweite Eingriffsstruktur (42) aufweist, die ein Paar entgegengesetzter Flächen (60, 62) aufweist, die durch eine Breite getrennt sind, und sich von der zweiten Eingriffsfläche (40) in einem zweiten schiefen Winkel um die Querachse (T) erstreckt und ein einziges Flanschelement oder Kiel aufweist, das/der sich im Wesentlichen quer über die zweite Eingriffsfläche (40) in einer Richtung im Wesentlichen quer zu der Längsachse (L) erstreckt und sich von der zweiten Eingriffsfläche (40) in einem zweiten schiefen Winkel um die Querachse (T) erstreckt, wobei die zweite Eingriffsstruktur (42) annähernd zentral entlang der Längsachse (L) angeordnet ist und für eine Anordnung innerhalb einer vorgeformten Öffnung in einer benachbarten Wirbelendplatte konfiguriert ist.

2. Die Prothesenvorrichtung (20) gemäß Anspruch 1, wobei die erste Eingriffsstruktur (34) im Wesentlichen parallel zu der zweiten Eingriffsstruktur (42) ist, wenn die erste Eingriffsfläche (32) im Wesentlichen parallel zu der zweiten Eingriffsfläche (40) ist.

3. Die Prothesenvorrichtung (20) gemäß Anspruch 2, wobei die erste Eingriffsstruktur (34) im Wesentlichen ähnlich wie die zweite Eingriffsstruktur (42) ist.

4. Die Prothesenvorrichtung (20) gemäß Anspruch 3, wobei das Paar entgegengesetzter Flächen (48, 50; 60, 62) von jeder der ersten und der zweiten Eingriffsstruktur (34, 42) im Wesentlichen parallel ist und sich entlang einem Großteil der Länge des Flanschelements erstreckt.

5. Die Prothesenvorrichtung (20) gemäß Anspruch 4, wobei die Breite (52, 64) des jeweiligen Flanschelements (34, 42) entlang der Länge des Flanschelements (34, 42) im Wesentlichen konstant ist.

6. Die Prothesenvorrichtung (20) gemäß Anspruch 4, wobei die Breite (52, 64) des jeweiligen Flanschelements (34, 42) an mindestens einem Ende des Flanschelements (34, 42) verjüngt ist.

7. Die Prothesenvorrichtung (20) gemäß Anspruch 6, wobei die erste und die zweite Eingriffsstruktur (34, 42) ferner mindestens eine Öffnung (54) aufweisen, die sich zwischen den korrespondierenden parallelen Flächen (48, 50, 60, 62) durch das korrespondierende Flanschelement hindurch erstreckt.

8. Eine Zwischenwirbel-Gelenk-Prothesenvorrichtung zur Anordnung innerhalb eines Raumes zwischen einem Paar von Wirbeln, die eine Längsachse (L), eine Querachse (T) und eine Vertikalachse (R) hat, wobei die Vorrichtung aufweist:
eine erste Komponente (80) für einen Eingriff mit dem ersten Wirbel, eine zweite Komponente für einen Eingriff mit dem zweiten Wirbel und einen Gelenk-Zentralabschnitt zwischen der ersten und der zweiten Komponente, mittels dessen die obere und die untere Komponente (22, 24) in bewegbarem Eingriff miteinander sind, wobei die erste Komponente (80) aufweist: eine erste Eingriffsfläche (82), die im Wesentlichen planar ist, und eine erste Eingriffsstruktur (84), die sich von der ersten Eingriffsfläche (82) aus erstreckt, wobei die erste Eingriffsstruktur (84) ein erstes Paar Flanschelemente (86, 88) aufweist und, entlang der Längsachse (L), annähernd zentral entlang der korrespondierenden Längsabmessung der Vorrichtung angeordnet ist, wobei jedes Flanschelement (86, 88) ein Paar entgegengesetzter Flächen (90, 92; 100, 102) aufweist, die durch eine Breite (94, 104) voneinander getrennt sind, und sich in einer Richtung im Wesentlichen entlang der Querachse (T) erstreckt und sich von der ersten Eingriffsfläche (82) in einem schiefen Winkel zwischen dem jeweiligen Flanschelement (86, 88) und der ersten Eingriffsfläche (82) erstreckt, so dass die Flanschelemente (86, 88) im Wesentlichen senkrecht zueinander sind, und wobei sich jedes der Flanschelemente (86, 88) im Wesentlichen quer über die erste Eingriffsfläche (82) erstreckt und die Flanschelemente (86, 88) für eine Anordnung innerhalb von vorgeformten Öffnungen in einer benachbarten Wirbelendplatte konfiguriert sind.

9. Die Prothesenvorrichtung (80) gemäß Anspruch 8, wobei das erste Paar von Flanschelementen (86, 88) im Wesentlichen symmetrisch zueinander um eine Ebene ist, die durch einen Mittelpunkt der ersten Eingriffsfläche (82) hindurchgeht und sich im Wesentlichen quer zu der ersten Eingriffsfläche (82) erstreckt.

10. Die Prothesenvorrichtung (80) gemäß Anspruch 9, wobei die Ebene auch im Wesentlichen quer zu der Länge der ersten Eingriffsfläche (82) ist.

11. Die Prothesenvorrichtung (80) gemäß Anspruch 10, wobei jedes von dem ersten Paar von Flanschelementen (86, 88) entlang seiner Länge eine im Wesentlichen konstante Breite hat.

12. Die Prothesenvorrichtung (80) gemäß Anspruch 11, wobei jedes von dem ersten Paar von Flanschelementen (86, 88) entlang seiner Länge eine im Wesentlichen konstante Höhe hat.

13. Die Prothesenvorrichtung (80) gemäß Anspruch 8, wobei die zweite Komponente aufweist:
eine zweite Eingriffsfläche und
eine zweite Eingriffsstruktur, die sich von der zweiten Eingriffsfläche aus erstreckt, wobei die zweite Eingriffsstruktur ein zweites Paar von Flanschelementen aufweist, wobei sich jedes Flanschelement von der zweiten Eingriffsfläche aus in einem schiefen Winkel erstreckt, so dass die Flanschelemente im Wesentlichen senkrecht zueinander sind.

14. Die Prothesenvorrichtung (80) gemäß Anspruch 13, wobei mindestens eines von dem zweiten Paar von Flanschelementen im Wesentlichen parallel zu einem von dem ersten Paar von Flanschelementen ist, wenn die zweite Eingriffsfläche im Wesentlichen parallel zu der ersten Eingriffsfläche ist.

15. Die Prothesenvorrichtung (80) gemäß Anspruch 14, wobei jedes von dem zweiten Paar von Flanschelementen im Wesentlichen parallel zu einem von dem ersten Paar von Flanschelementen ist, wenn die zweite Eingriffsfläche im Wesentlichen parallel zu der ersten Eingriffsfläche ist.

## Revendications

1. Un dispositif de prothèse articulaire intervertébrale (20) présentant un axe longitudinal (L), un axe transversal (T) et un axe vertical (R), et comprenant :
un composant supérieur (22), un composant inférieur (24) et une partie centrale articulaire (26) entre les composants supérieur et inférieur (22, 24), moyennant laquelle les composants supérieur et inférieur (22, 24) sont en prise entre eux de façon mobile, le composant supérieur (22) présentant une première surface de prise (32) et une première structure de prise (34) présentant une paire de surfaces opposées (48, 50), séparées par une largeur (52), et s'étendant à partir de la première surface de prise (32) selon un premier angle oblique entre la première structure de prise (34) et la première surface de prise (32), et comprenant un seul élément de bride ou quille s'étendant essentiellement en travers la première surface de prise (32) dans une direction essentiellement le long de l'axe transversal (T), la première structure de prise (34), le long de l'axe longitudinal (L), étant située approximativement au centre le long de la dimension longitudinale correspondante du dispositif (20) et étant configurée pour une disposition dans une ouverture préformée dans une plaque terminale vertébrale adjacente ; et le composant inférieur (24) présentant une deuxième surface de prise (40) et une deuxième structure de prise (42) présentant une paire de surfaces opposées (60, 62) séparées par une largeur, et s'étendant à partir de la deuxième surface de prise (40) selon un deuxième angle oblique autour de l'axe transversal (T) et comprenant un seul élément de bride ou quille s'étendant essentiellement en travers la deuxième surface de prise (40) dans une direction essentiellement transversale à l'axe longitudinal (L) et s'étendant à partir de la deuxième surface de prise (40) selon un deuxième angle oblique autour de l'axe transversal (T), la deuxième structure de prise (42) étant située approximativement au centre le long de l'axe longitudinal (L) et étant configurée pour une disposition dans une ouverture préformée dans une plaque terminale vertébrale adjacente.

2. Le dispositif de prothèse (20) selon la revendication 1, dans lequel la première structure de prise (34) est essentiellement parallèle à la deuxième structure de prise (42), lorsque la première surface de prise (32) est essentiellement parallèle à la deuxième surface de prise (40) .

3. Le dispositif de prothèse (20) selon la revendication 2, dans lequel la première structure de prise (34) est essentiellement similaire à la deuxième structure de prise (42) .

4. Le dispositif de prothèse (20) selon la revendication 3, dans lequel la paire de surfaces opposées (48, 50 ; 60, 62) de chacune des première et deuxième structures de prise (34, 42) sont essentiellement parallèles et s'étendent le long d'une majorité de la longueur de l'élément de bride.

5. Le dispositif de prothèse (20) selon la revendication 4, dans lequel la largeur (52, 64) de l'élément de bride (34, 42) respectif est essentiellement constante le long de la longueur de l'élément de bride (34, 42).

6. Le dispositif de prothèse (20) selon la revendication 4, dans lequel la largeur (52, 64) de l'élément de bride (34, 42) respectif est conique à au moins une extrémité de l'élément de bride (34, 42).

7. Le dispositif de prothèse (20) selon la revendication 6, dans lequel les première et deuxième structures de prise (34, 42) comprennent en outre au moins une ouverture (54) s'étendant à travers l'élément de bride correspondant entre les surfaces parallèles correspondantes (48, 50 ; 60, 62).

8. Un dispositif de prothèse articulaire intervertébrale pour disposition dans un espace entre une paire de vertèbres, présentant un axe longitudinal (L), un axe transversal (T) et un axe vertical (R), le dispositif comprenant :
un premier composant (80) pour se mettre en prise avec la première vertèbre, un deuxième composant pour se mettre en prise avec la deuxième vertèbre, et une partie centrale articulaire entre les premier et deuxième composants, moyennant laquelle les composants supérieur et inférieur (22, 24) sont en prise entre eux de façon mobile, le premier composant (80) présentant : une première surface de prise (82) qui est essentiellement plane, et une première structure de prise (84) s'étendant à partir de la première surface de prise (82), la première structure de prise (84) comprenant une première paire d'éléments de bride (86, 88) et, le long de l'axe longitudinal (L), étant située approximativement au centre le long de la dimension longitudinale correspondante du dispositif, chaque élément de bride (86, 88) présentant une paire de surfaces opposées (90, 92 ; 100, 102) qui sont séparées par une largeur (94, 104), et s'étendant dans une direction essentiellement le long de l'axe transversal (T) et s'étendant à partir de la première surface de prise (82) selon un angle oblique entre l'élément de bride (86, 88) respectif et la première surface de prise (82), de sorte que les éléments de bride (86, 88) sont essentiellement perpendiculaires l'un à l'autre, et chacun des éléments de bride (86, 88) s'étend essentiellement en travers la première surface de prise (82) et les éléments de bride (86, 88) sont configurés pour une disposition dans des ouvertures préformées dans une plaque terminale vertébrale adjacente.

9. Le dispositif de prothèse (80) selon la revendication 8, dans lequel la première paire d'éléments de bride (86, 88) sont essentiellement symétriques l'un à l'autre autour d'un plan passant à travers un point central de la première surface de prise (82) et s'étendant essentiellement transversal à la première surface de prise (82).

10. Le dispositif de prothèse (80) selon la revendication 9, dans lequel le plan est également essentiellement transversal à la longueur de la première surface de prise (82).

11. Le dispositif de prothèse (80) selon la revendication 10, dans lequel chacun de la première paire d'éléments de bride (86, 88) présente une largeur essentiellement constante le long de sa longueur.

12. Le dispositif de prothèse (80) selon la revendication 11, dans lequel chacun de la première paire d'éléments de bride (86, 88) présente une hauteur essentiellement constante le long de sa longueur.

13. Le dispositif de prothèse (80) selon la revendication 8, dans lequel le deuxième composant comprend :
une deuxième surface de prise et
une deuxième structure de prise s'étendant à partir de la deuxième surface de prise, la deuxième structure de prise comprenant une deuxième paire d'éléments de bride, chaque élément de bride s'étendant à partir de la deuxième surface de prise selon un angle oblique, de sorte que les éléments de bride sont essentiellement perpendiculaires l'un à l'autre.

14. Le dispositif de prothèse (80) selon la revendication 13, dans lequel au moins un de la deuxième paire d'éléments de bride est essentiellement parallèle à un de la première paire d'éléments de bride lorsque la deuxième surface de prise est essentiellement parallèle à la première surface de prise.

15. Le dispositif de prothèse (80) selon la revendication 14, dans lequel chacun de la deuxième parie d'éléments de bride est essentiellement parallèle à un de la première paire d'éléments de bride lorsque la deuxième surface de prise est essentiellement parallèle à la première surface de prise.
